# EUROPEAN PATENT SPECIFICATION

(11) **EP 3 197 906 B1**
(45) Date of publication and mention of the grant of the patent: **25.10.2023**
(21) Application number: 14780807.5
(22) Date of filing: 26.09.2014
(51) Int. Cl.: C07F 11/00, C07C 311/09, B01J 23/00, B01J 23/89, H01M 8/10, H01M 8/1025, H01M 8/103, H01M 8/1048, H01M 8/1086, B01J 23/30, B01J 27/188, B01J 35/00, H01M 8/1027, H01M 8/1081

(54) **PROCESSES FOR PREPARING POLYOXOMETALATE SALTS FOR USE IN PROTON EXCHANGE MEMBRANES AND FUEL CELLS**
VERFAHREN ZUR HERSTELLUNG VON POLYOXOMETALAT-SALZEN ZUR VERWENDUNG IN PROTONEN-AUSTAUSCH-MEMBRANEN UND BRENNSTOFFZELLEN
PROCÉDÉS DE PREPARATION DE SELS DE POLYOXOMETALATE A UTILISER DANS DES MEMBRANES ECHANGEUSES DE PROTONS ET DES PILES A COMBUSTIBLE

(43) Date of publication of application: 02.08.2017
(73) Proprietor: Mebius d.o.o., 1000 Ljubljana (SI)
(72) Inventor: HOCEVAR, Stanko, SI-1000 Ljubljana (SI); KRZAN, Andrej, SI-1000 Ljubljana (SI)
(74) Representative: Mewburn Ellis LLP
(86) International application number: PCT/EP2014/070697
(87) International publication number: WO 2016/045754

(56) References cited:
- WO-A1-2006/080766
- WO-A2-2009/108222
- CN-A- 101 219 995
- CN-A- 101 219 995
- CN-A- 103 288 739
- CN-A- 103 333 114
- CN-A- 103 665 369
- CN-A- 103 666 536
- CN-A- 103 922 893
- US-A- 6 059 943
- US-A1- 2013 085 191
- HAI-JUAN DU ET AL: "Templated fabrication, isomer recognition of series of 1,1'-(alkane-1,[omega]-diyl)-bis(3-methyli midazolium)-induced polyoxometalates ([omega]=1-11)", INORGANICA CHIMICA ACTA, vol. 409, 7 October 2013 (2013-10-07), pages 418-426, XP055196874, ISSN: 0020-1693, DOI: 10.1016/j.ica.2013.09.050
- ATHANASIOS B. BOURLINOS ET AL: "A Liquid Derivative of 12-Tungstophosphoric Acid with Unusually High Conductivity", JOURNAL OF THE AMERICAN CHEMICAL SOCIETY, vol. 126, no. 47, 2004, pages 15358-15359, XP055196859, ISSN: 0002-7863, DOI: 10.1021/ja046821b
- NAIQIN TIAN ET AL: "Synthesis and conductivity of hybrid materials based on germanium-containing polyoxometalates and ionic liquids", JOURNAL OF COORDINATION CHEMISTRY, vol. 66, no. 3, February 2013 (2013-02), pages 379-384, XP055196880, ISSN: 0095-8972, DOI: 10.1080/00958972.2012.757601

## Description

### Technical field

The present invention relates to a process for the preparation of an organic-inorganic hybrid compound.

### Background of the Invention

The operation of electrochemical devices requires the occurrence of oxidation and reduction reactions that are physically separated into compartments connected through conductors of charge carriers, which may be either electrons or ions. The two compartments are most commonly connected through an external electron conductor which conducts electrical current produced or consumed in the reactions, whereas electrolytes conduct ionic species between the two compartments and complete the electrochemical circuit needed for operation. A particular case of an ionically conducting electrolyte is a proton conducting electrolyte which is employed in devices such as fuel cells which require proton conduction in part of the electrochemical circuit. For electrolytes to perform their role they must be poor conductors of electrons.

Proton conducting electrolytes are commonly manufactured in the form of solid electrolytes as thin membranes known as proton exchange membranes (PEM). The advantages of using PEM in electrochemical devices such as fuel cells are numerous. A solid electrolyte is simpler and more compact than liquid electrolytes, has better mechanical properties that allow for thin membranes, and supports convenient manufacturing methods.

When using a PEM as an electrolyte in a fuel cell numerous properties are desired. These include: high ionic (such as proton) conductivity; zero conductivity of electrons; low gas and fuel permeability; resistance to swelling; minimal water transport; high resistance to dehydration, oxidation, reduction and hydrolysis; a high ion transport number; surface properties allowing easy catalyst bonding; and mechanical strength. In order to meet these requirements PEM usually consist of a polymer matrix containing functional groups or components capable of supporting the required properties.

The most widely and commercially used polymers for PEM manufacturing belong to a class of compounds known as perfluorosulfonic acids (PFSA) developed at DuPont and Dow Chemicals Company. These polymers are fully fluorinated, meaning that all sites that would be occupied by hydrogen atoms in a conventional hydrocarbon polymer have been replaced by fluorine atoms. This makes the polymers extremely resistant to chemical attack. The additional presence of sulfonic groups results in a specific morphology that supports proton conductivity.

PFSA polymers are generally synthesized by copolymerization of a derivatized, or active, comonomer with tetrafluoroethylene (TFE). After synthesis, the thermoplastic, hydrophobic and electrochemically inert polymer is converted into an ionomer in its salt form through basic hydrolysis. This can then be converted to the protonated form by ion-exchange with a strong acid, with the sulfonate groups (RSO₃⁻) acting as the stationary counter charge to the mobile protons (H⁺).

[K.D. Kreuer, On the development of proton conducting polymer membranes for hydrogen and methanol fuel cells, Journal of Membrane Science 185 (2001) 29-39.]

[M. H. D. Othman, A. F. Ismail, A. Mustafa, Recent Development of Polymer Electrolyte Membranes for Direct Methanol Fuel Cell Application -A Review, Malaysian Polymer Journal, Vol. 5, No. 2, p 1- 36, 2010. Available online at www.fkkksa.utm.my/mpj.]

Another type of polymer used for PEM in fuel cells is a derivatized trifluorostyrene (TFS) of the type developed by Ballard Advanced Materials. This polymer has a fully fluorinated backbone, but some of the side chains have hydrogen atoms. The polymer is synthesized by copolymerizing derivatized and non-derivitized trifluorostyrene monomers resulting in an electrochemically inactive thermoplastic. Non-derivatized monomers are then sulfonated to yield a proton conducting polymer.

S. M. Javaid Zaidi, Research Trends in Polymer Electrolyte Membranes for PEMFC, Chapter 2 in S.M.J. Zaidi, T. Matsuura (eds.) Polymer Membranes for Fuel Cells, 7 doi: 10.1007/978-0-387-73532- 0, © Springer Science+ Business Media, LLC 2009, pp. 7-25; Wei, J., Stone, C., Steck, A.E.: Trifluorostyrene and substituted trifluorostyrene copolymeric compositions and ion-exchange membranes formed therefrom.

5,422,411, United States (1995); Beattie, P.D., Orfino, F.P., Basura, V.I., Zychowska, K., Ding, J., Chuy, C., Schmeisser, J., Holdcraft, S., Ionic conductivity of proton exchange membranes, Journal of Electroanalytical Chemistry, 503: 45-56 (2001); Basura, V.I., Chuy, C., Beattie, P.D., Holdcraft, S., Effect of equivalent weight on electrochemical mass transport properties of oxygen in proton exchange membranes based on sulfonated α,β,β-trifluorostyrene (BAM®) and sulfonated styrene-(ethylenebutylene)- styrene triblock (DAIS-analytical) copolymers, Journal of Electroanalytical Chemistry, 501: 77-88 (2001).

WO 2006/080766 A1 (UNIV INDUSTRY UNIVERSITY COOPE [KR]; LEE YOUNG-MOO [KR]; LEE CHANG-HYU) 3 August 2006 (2006-08-03)

CN 103 665 369 A (LANZHOU CHEM PHYS INST) 26 March 2014 (2014-03-26)

HAI-JUAN DU ET AL: "Templated fabrication, isomer recognition of series of 1, 1 '-(alkane-1,[omega]-diyl) bis(3-methylimidazolium)-inducedpolyoxometalates ([omega]=1-11 )",

INORGANICA CHIMICA ACTA,vol. 409, 7 October 2013 (2013-10-07), pages 418-426, XP055196874,ISSN: 0020-1693, DOl: 10.1016/j.ica.2013.09.050

CN 103 666 536 A (UNIV LIAONING PET CHEM TECH) 26 March 2014 (2014-03-26)

US 2013/085191 A1 (LASKOSKI MATTHEW [US]) 4 April 2013 (2013-04-04)

ATHANASIOS B. BOURLINOS ET AL: "A Liquid Derivative of 12-TungstophosphoricAcid with Unusually High Conductivity", JOURNAL OF THE AMERICAN CHEMICAL SOCIETY, vol. 126, no. 47, 2004, pages 15358-15359, XP055196859, ISSN: 0002-7863, DOI: 10.1021/ja046821b

CN 103 333 114 A (UNIV QINGDAO SCIENCE & TECH) 2 October 2013 (2013-10-02)

CN 101 219 995 A (UNIV EAST CHINA SCIENCE & TECH [CN]) 16 July 2008 (2008-07-16)

NAIQIN TIAN ET AL: "Synthesis and conductivity of hybrid materials based on germanium-containing polyoxometalates and ionic liquids", JOURNAL OF COORDINATION CHEMISTRY, vol. 66, no. 3, February 2013 (2013-02), pages 379-384, XP055196880, ISSN: 0095-8972, DOI: 10.1080/00958972.2012.757601

WO 2009/108222 A2 (3M INNOVATIVE PROPERTIES CO [US]; COLORADO SCHOOL OF MINES [US]) 3 September 2009 (2009-09-03)

CN 103 288 739 A (UNIV SHAANXI NORMAL) 11 September 2013 (2013-09-11)

CN 103 922 893 A (UNIV QINGDAO SCIENCE & TECH) 16 July 2014 (2014-07-16)

US 6 059 943 A (MURPHY OLIVER J [US] ET AL) 9 May 2000 (2000-05-09)

CN 101 219 995 A (UNIV EAST CHINA SCIENCE & TECH [CN]) 16 July 2008 (2008-07-16)

Other homogeneous proton conducting polymers are also known. They generally consist of a variety of organic polymeric structures derivatized with sulfonic groups. Sulfonated polymers can have poor physical properties making them difficult to handle or, for example, can be susceptible to tearing or puncturing.

PEM manufactured from sulfonated polymers exhibit proton conductivities suitable for fuel cell operation when they are well hydrated, whereas in anhydrous form they exhibit negligible proton conductivities. High hydration levels generally result in significant swelling of the membrane. At ambient pressures and temperatures approaching the boiling point of water the hydration level of sulfonated polymers starts to decrease resulting in a substantial decrease in proton conductivity. The loss of water from the system renders the sulfonated polymer based PEM-containing device non- operational at temperatures between 80°C and 110°C. The temperature at which the PEM loses conductivity may be partially overcome by operation of the device at higher pressures or by incorporating hydrophilic components such as silica in the membrane. However, both solutions have serious limitations. In some applications, humidifying incoming reactant gas streams has been practiced in order to elevate the level of hydration in the PEM, however, humidifiers add to the cost and complexity of the system and increase parasitic power losses during operation. Humidifying becomes increasingly difficult at elevated temperatures. As a result, there is a need for proton- conducting materials that are capable of efficient proton transport with little or, ideally, no water.

In some applications, including some automotive applications, there is a desire to operate fuel cells at higher temperatures, i.e. above 100°C, preferably at temperatures above 135 °c or even in the range from 200°C to 300°C. Operation at elevated temperatures can simplify the cooling system while improving heat management and may result in higher efficiency of electrochemical reactions and possibly the use of the generated waste heat in combined heat and power systems. Operating at higher temperatures may also improve catalyst resistance to carbon monoxide poisoning when using reformed fuels or allow the use of non-noble metal electrode catalysts, thus reducing costs.

An alternate approach to developing homogeneous proton conducting membranes is to combine an electrochemically active or inactive polymer with a proton conductive additive or filler other than water. An example of such non-homogeneous, composite membranes is offered by membranes based on polybenzimidazole copolymers in which phosphoric acid is used as the filler to produce membranes capable of operating at temperatures up to 180°C without the need for humidification. A limitation of this particular solution is the tendency of phosphoric acid to leach from the membrane at lowoperating temperatures.

Among potential proton conducting polymer fillers one can also find heteropolyacids (HPA), which are characterized by some of the highest proton conductivities. These strongly hydrated inorganic compounds suffer from their high solubility in water, which can result in substantial leaching during fuel cell operation. Strategies for integration of HPA therefore focus on methods for anchoring HPA in polymer matrices. A comprehensive review of the state-of-the-art in the use of HPA in proton exchange fuel cells was recently published by Sachdeva et al. (Struct. Bond. 2011, 141, 115-168). Strategies to employ HPA in efficient PEM may be summarized into the following classes: a) HPA doped membranes, b) sol-gel based HPA membranes, and c) polypom membranes.

The current state-of-the-art concerning the use of HPA derivatives in fuel cells can be seen from recent literature.

US 6,864,006, US 6,680,138 and US 2004 0028978 (Honma et al.) propose proton conducting membranes employing ion conductors composed of heteropolyacids as a proton conducting agent in conjunction with reagents containing hydrolysable silyl groups and amino groups. Bourlinos et al. (J. Am. Chem. Soc. 2004, 126, 15358-15359) report on polyoxometalates-based liquid salts containing quaternary ammonium cations that give medium ionic conductivity of 6 x 10⁻⁴ S/cm at 140°C and are proposed as an alternative for attaining anhydrous proton conduction. EP1685618, (Krzan and Hocevar) proposes composite membranes that use a variety of electrochemically inert high- performance polymers with high chemical stability blended with ionic liquids, heteropolyacids and silica to give membranes of medium conductivity. US 8,206,874, (Hamrock et al.) proposes PEM containing polyoxometalates or heteropolyacids comprising transition metals Mn or Ce. Synthesized lacunary HPA were used to prepare organic-inorganic hybrid monomers that can be polymerized in a radical reaction to yield a polymer electrolyte. PEM and MEA were produced when these polymer electrolytes were combined with polymers that could be fluorinated and could contain acidic groups.

Ionic liquids, i.e. salts with low melting temperatures, generally below 100°C, are known to exhibit high ionic conductivity in the absence of water or solvents, high thermal stability, and a very low vapor pressure. Structurally, ionic liquids are salts comprised of organic cations, of which quaternary ammonium cations, N-substituted quaternary imidazolium cations, and quaternary pyridinium cations are most common. The charge is normally balanced by anions that are conjugate bases of strong acids.

These materials have also been explored as electrolytes for batteries and other devices, and also as ion conductors in PEM. Several proposals have been made for the use of ionic liquids as proton exchange materials mainly intended for use in fuel cells, which include the use of aprotic and protic ionic liquids. The proton conductivity cited in the examples was in the range of at most 10⁻⁴ to 10⁻³ S/cm without humidification thus necessitating a further improvement for utilization in PEM without humidification. In particular, ionic liquids were used to enhance proton conductivity in polymer blends.

When blended with a polymer the ionic liquid can also act as a plasticizer or partial solvent at elevated temperatures. An example of the approach, combining a perfluorosulfonic polymer with ionic liquids is offered by Schmidt et al. (Chem. Eng. Technol. 2008, 31, 13-22).

However, materials prepared by impregnating a polymer material with an ionic liquid, suffer from a latent problem due to the leakage of the unbound ionic liquid. To address this problem, solid electrolytes were developed by increasing the molar mass of the ionic liquid. An example of this approach is the polymerization of a protic ionic liquid, a N-vinylimidazole salt, however, the proton conductivity of the resulting polymer was not at a satisfactory level and needed to be further improved. A review of polymeric ionic liquids and their applications was published by Mecerreyes (Prag. Poly. Sci. 2011, 36, 1629-1648).

A proton conducting material and fuel cell using the same is presented by Hojo et al. in US 8,211,590,
wherein stable fuel cell functioning is alleged without humidification in the temperature range between room temperature to about 200°C. The proton conductivity is supported by heterocyclic organic compounds based on imidazole derivatives.

Honma et al. (Electrochimica Acta 2008, 53, 7638) described an organic-inorganic crystalline electrolyte based on 12-phosphotungstic acid and an ionic liquid. The hybrid material was shown to be thermally stable up to 400°C and exhibited a high ion conductivity jump due to melting. At 240°C a conductivity of 1 × 10⁻³ S/cm was reported. Similar hybrids with low melting temperatures were prepared by Rickert et al. (J. Phys. Chem. B 2007, 111, 4685-4692) by pairing Keggin and Lindquist polyoxometalate anions with tetraalkylphosphonium cations.

The use of ionic liquids in proton exchange electrolytes shows promise due to inherent ion conductivity in the absence of water, however improved solutions that could support commercially interesting products are still being sought.

Rajkumar and Rao (J. Chem Sci. 120, 587-594, 2008; Mat. Chem. Phys. 112, 853-857, 2008; Solid State Sci. 11, 36-42, 2009) reported on the synthesis and characterization of molecular hybrid materials prepared by reacting 1-butyl-3-methyl immidazolium bromide - an ionic liquid with phosphotungstic, silicotungstic and phosphomolibdic acids. Ammam *et al.* (J. Elchem.

Soc. 158, A14- A21, 2011) used two of the named hybrid salts for electrophoretic deposition and investigated capacitance and electrical cyclability properties.

While the methods outlined above allegedly allow the fabrication of composite membranes that may present enhanced structural and thermal stability and ionic conductivity, the methods used do not allow the flexibility needed for fabricating composite membranes suitable for use in a wide range of applications. Thus there is a continuing need to look for electrolytes, proton exchange membranes, and membrane fabricating processes that allow greater flexibility in controlling the physical properties of the composite membranes.

### General Notes

A number of patents and publications are cited herein in order to more fully describe and disclose the invention and the state of the art to which the invention pertains.

Throughout this specification, including the claims which follow, unless the context requires otherwise, the word "comprise" and variations such as "comprises" and "comprising," will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise.

Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment.

This disclosure includes information that may be useful in understanding the present invention. It is not an admission that any of the information provided herein is prior art or relevant to the presently claimed invention, or that any publication specifically or implicitly referenced is prior art.

### Summary of the Invention

It is an objective of the present invention to provide a process for preparing an organic-inorganic compound comprising the step of mixing a heteropolyacid with an ionic liquid comprising an organic cation and a counter anion to perform a metathesis reaction. The organic-inorganic compound may be used in an improved proton-conducting membrane and, more particularly, may be used in an improved method of fabricating said membranes and to provide composite membranes obtainable using such processes. In particular, the present invention seeks to improve on the current state-of-the-art by providing proton conducting electrolytes and proton exchange membranes that are capable of supporting fuel cell operation at temperatures between 195 and 300°C under operating conditions that do not include any form of humidification of the reagents or device.

### Heteropolyoxometalate Hybrid Compound

Described herein is an organic-inorganic hybrid compound comprising a salt of an organic cation with a heteropolyoxometalate anion, comprising a stoichiometric ratio of organic cation to heteropolyoxometalate anion.

The organic cation and the heteropolyoxometalate anion have a stoichiometric ratio. "Stoichiometric ratio" refers to a ratio of organic cation to heteropolyoxometalate anion which is charge-balanced.

For example, where the charge on the anion is (4) and the charge on the cation is (1⁺), a stoichiometric molar ratio of cations to anions would be 4:1. This means that the hybrid compound is free of impurities which would upset the charge balance resulting in a non-stoichiometric ratio of organic cation to heteropolyoxometalate anion. The ionic salt is therefore of very high purity.

The stoichiometric compounds of the present invention display properties which make them suitable for use in fuel cell electrolytes such as proton exchange membranes. For example, the organic- inorganic hybrid compound is insoluble in water, which is crucial to the successful application of the compound in fuel cells where water is a common by-product. Furthermore, the compound is not hygroscopic and has a very high thermal stability. This allows the compound to be used in a proton conducting electrolyte which is operational between temperatures of 195°C and 300°C, which provides various advantages as described above.

The stoichiometric hybrid compounds of the present invention suitably exhibit a second-order phase transition at high temperature which leads to an ionic conductivity of between 10⁻³ S/cm and 10⁻² S/cm which is sustainable across the whole temperature range of 195°C to 300°C. Such a high ionic conductivity at high temperature means that the compounds may be used to provide highly efficient proton exchange membranes and therefore highly efficient fuel cells.

In the organic-inorganic hybrid compound, the organic cation may be selected from one or more of substituted or unsubstituted imidazolium, substituted or unsubstituted pyridinium, quaternary ammonium, substituted or unsubstituted piperidinium and tetraalkyl phosphonium.

In the organic-inorganic hybrid compound, the organic cation may be defined according to any one of the following formulae: 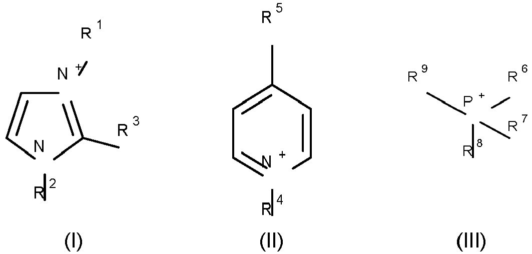 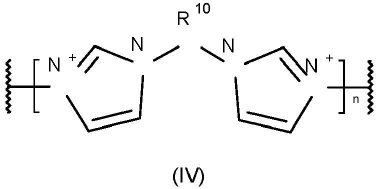 wherein R¹ and R² are each independently saturated aliphatic C1-4alkyl; R³ is independently hydrogen, or linear or branched C1-4alkyl; and R⁴ and R⁵ are each independently saturated aliphatic C1-4alkyl; R⁶, R⁷, R⁸ and R⁹ are each independently selected from linear or branched Cs-1salkyl; R¹⁰ is independently selected from aryl, heteroaryl, cycloaliphatic, alkenyl and alkynyl; and n is an integer within the range from 5 to 1000. Alkyenyl and alkynyl groups may suitably be rigid.

Suitable R¹⁰ groups include optionally substituted or unsubstituted ethenyl, ethynyl, aryl, heteroaryl or cycloalkyl linker groups, e.g. -CH=CH- (cis or trans), phenylene (e.g. 1,4- or 1,3-phenylene) or pyridylene (e.g. 2,6-); the degree of polymerisation - n, of the polymeric salt may be sufficiently large that the salt is insoluble in water. The degree of polymerisation -n is an integer within the range from 5 to 1000. (see J.Y. Ying, Y. Zhang, D. Hu, P.K. Patra, Polymeric salts and poly-NHC-metal complexes, US 8,163,851 B2).

In some embodiments, the organic cation is not 1-butyl-3-methylimidazolium.

Such cations or quaternary pyridinium cations (Py) provide formation of water insoluble salts with polyoxometalate anions that exhibit substantial proton conductivity within the range of around 1 to around 50 mS/cm at elevated (below 100 °C) or high temperatures (over 100 °c and up to 300 °C). Proton transport between the protonated Py or Im (HPy⁺ or Hlm⁺) and neat Py or Im (the proton defects, i.e., vacant nitrogen site of Py or Im) plays an important role for intermolecular proton transfer, and at an optimum composition enhances the proton conduction by Grotthuss mechanism (see Kreuer, K. D.; Fuchs, A.; Ise, M.; Spaeth, M.; Maier, J. Electrochim. Acta 1998, 43, 1281).

The structure of 1-butyl-3-methylimidazolium (BMIM) is as follows:

| **Name** | **Abbreviation** | **Structure** |
|---|---|---|
| 1-Butyl-3-methylimidazolium | BMIM | 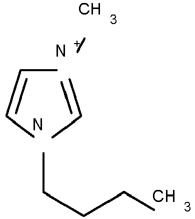 |

The organic cation may be selected from DMIM, EMIM, TMIM, NBMP, THTDP and PIM.

The full IUPAC names and structures of these organic cations is as follows:

| **Name** | **Abbreviation** | **Structure** |
|---|---|---|
| 1,3-Dimethylimidazolium | DMIM | 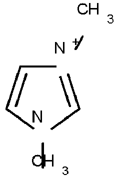 |
| 1,2,3-Trimethylimidazolium | TMIM | 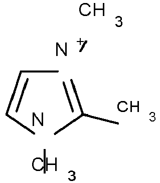 |
| 1-Ethyl-3-methylimidazolium | EMIM | 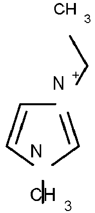 |
| N-Butyl-4-methylpyridinium (1-Butyl-4-methylpyridinium) | NBMP | 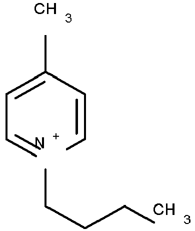 |
| Trihexyltetradecylphos phonium | THTDP | 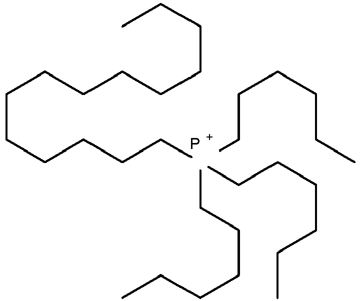 |
| Polyimidazolium | PIM | 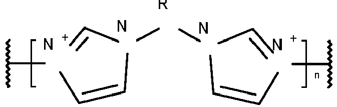 n = 5-1000 |

These particular cations provide salts when reacted with polyoxometalate anions which have a higher stability and exhibit an increased ionic conductivity. Furthermore, these salts are insoluble in water, which reduces the risk of leaching of the salt from a proton exchange membrane during use.

The polyoxometalate anion may be an isopolyoxometalate or a heteropolyoxometalate comprising a transition metal atom selected from W, V, Mo, Nb, Mn and Ce.

This includes a wide range of polyoxometalate structures that have different physico-chemical properties and may or may not be centrosymmetric. This class of superacids allows for a change of anion charge (most commonly 3⁻, 4⁻ and 5⁻) and symmetry by the choice of metal ions in their structures. In turn these properties define the number of interacting 1+ charged organic cations and their distribution around the anion and consequently the molecular dynamics that governs conductivity. The polyoxometalate anions differ from other types of anions hitherto used in ionic liquids by their large size, which is in general larger than 1 nm. The centrosymmetric character of some polyoxoxmetalate anions leads to delocalization of negative charge over the surface of this relatively large structure.

The heteropolyoxometalate anion may be selected from silicotungstate and phosphotungstate. These two anions are among the most common and widely used heteropolyoxometalate anions, especially the phosphotungstate anion the synthesis of which is relatively simple starting from tungsten oxide and phosphoric acid. Besides, these two anions are centrosymmetric with negligible polarity, which renders them soluble when in the form of acids or salts in non-polar organic solvents.

Silicotungstate has the formula [W₁₂SiO₄₀]⁴⁻ and may be more conveniently denoted [SiW]⁴⁻. Phosphotungstate has the formula [PW₁₂O₄₀]³⁻and may be more conveniently denoted [PW]³⁻

### Method of Making the Heterooolvoxometalate Hybrid Compound

The present invention relates to a method of making an organic-inorganic hybrid compound comprising the stepof, mixing a heteropolyacid with an ionic liquid comprising an organic cation and a counter anion, wherein the organic-inorganic hybrid compound formed comprises a stoichiometric ratio of organic cation to heteropolyoxometalate anion; the organic cation is selected from 1,3-dimethyllimidazolium (DMIM), 1-ethyl-3-methylimidazolium (EMIM), 1,2,3-trimethylimidazolium (TMIM), N-butyl-4-methylpyridinium (NBMP), trihexyltetradecylphosphonium (THTDP) and polyimidazolium (PIM), wherein the number of repeat units in polyimidazolium is 5 and 1000; and the heteropolyoxometalate anion is selected from silicotungstate and phosphotungstate.

The term "organic salt" refers to a salt containing an organic cation. The identity of the anion is not particularly limited.

The organic salt may be an ionic liquid. Ionic liquids generally possess low melting points. The ionic liquid may be miscible in water.

The method may be carried out by mixing the heteropolyacid and organic salt reactants in their pure form. Alternatively, they may be present in solution or in suspension.

Preferably, the heteropolyacid is a stronger acid than the acid of which the counter anion is a conjugate base. This results in an efficient ion exchange reaction wherein the conjugate base of the HPA replaces the conjugate base of the organic salt to produce a stoichiometric (charge balanced) organic-inorganic hybrid compound.

Heteropolyacids (HPA) are compounds made up of a metal, oxygen, a p-block element and acidic hydrogen atoms. Polyoxometalate anions are the conjugate bases of HPAs, i.e.

HPAs undergo deprotonation to form polyoxometalate anions.

This general ion exchange reaction produces an organic-inorganic hybrid compound of stoichiometric composition; that is the combined charge of the organic cations matches exactly the charge of the polyoxometalate anion, regardless of any excess of reagent used.

The HPA is preferably silicotungstic acid (H₄W₁₂SiO₄₀) or phosphotungstic acid (H₃PW₁₂O₄₀).

The reaction product may be washed with a solvent in which the organic-inorganic hybrid compound is insoluble, but in which other species present in the reaction mixture are soluble.

The washing solvent may be water. The organic-inorganic hybrid compound formed in the method is generally insoluble in water, so a further step of washing with water removes impurities such as residual unreacted reactants to leave the pure stoichiometric product.

The further step of recrystallization of the organic-inorganic hybrid compound may be performed.

The organic cation may be selected from DMIM, EMIM, TMIM, NBMP, THTDP and PIM. The counter anion in the organic salt may be chloride, bromide, iodide, tetrafluoroborate, hexafluorophosphate, trifluoroacetate, methylsulfate, trifluoromethanesulfonate or trifluoromethanesulfonimide.

The organic salt may be insoluble in water.

A further step of activation may be carried out alongside the existing method steps. The activation step may include heating and/or ultrasonic mixing.

### Brief Description of the Drawings

Figure 1 shows the mass balance of the ion exchange reaction between silicotungstic acid and an imidazolium chloride ionic liquid carried out in distilled water at different ionic liquid additions to a constant quantity of the HPA. Open squares depict the mass of reagents remaining in solution and filled squares depict the mass of the insoluble reaction product.
Figure 2 shows the crystal structure of the ion pair formed by the reaction of phosphotungstic acid hydrate and 1-butyl-3-methyl imidazolium hexafluorophosphate (BMIMPFs). The 3 negative charge of the phosphotungstate anion is balanced by 3 imidazolium cations each carrying a 1⁺charge.
Figure 3 shows a thermal gravimetric analysis scan giving the relative mass loss in dependence of temperature for a representative organic-inorganic ion pair made by reacting silicotungstic acid and BMIMCI. The ion pair shows stability past 376°C while the differential thermal analysis plot shows a transition at 194°C.
Figure 4 shows a representative conductivity vs. reciprocal temperature diagram obtained from impedance spectroscopy measurements in dry helium of an organic-inorganic ion made by reacting silicotungstic acid and BMIMCI. The diagram indicates that conductivities above 10-³ S/cm are achieved above the temperature of the second order transition and that a hysteresis takes place under the used measurement conditions.
Figure 5 shows a representative differential scanning calorimetry (DSC) scan with two heating curves and one cooling curve for the ion pair formed from silicotungstic acid and BMIMCI. The peaks mark heat flows attributed to a reversible second order transition.

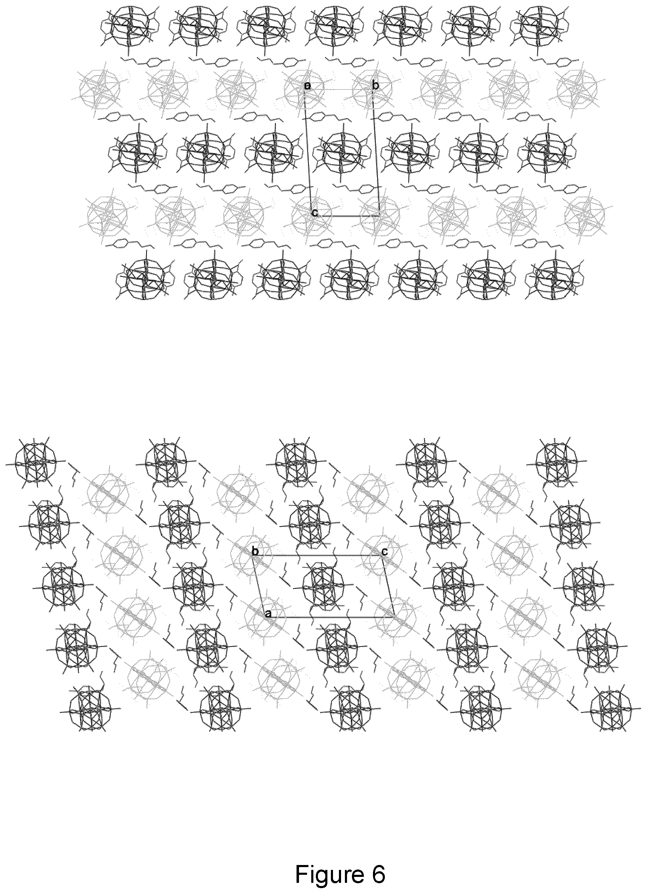

Figure 6 shows (a) view along the a-axis of the structure of the salt produced in Example 7, as determined by single-crystal X-ray crystallography, and (b) a view of the crystalline structure of the same salt along the b-axis.
Figure 7 shows a view of the same crystalline structure as shown in Figure 6, but along the c-axis.

### Detailed Description

Any sub-titles herein are included for convenience only, and are not to be construed as limiting the disclosure in any way.

The invention will now be further described with reference to the following non-limiting Figures and Examples. Other embodiments of the invention will occur to those skilled in the art in the light of these.

The organic-inorganic hybrid compounds described herein comprise a stoichiometric ratio of organic cation to heteropolyoxometalate anion. In other words, the compounds are of high purity, with little or no contamination within the ionic structure of the salt. Proton exchange membranes including such compounds are operational within a temperature range of 195-300°C, and it is therefore important that the polyoxometalate salt is stable and exhibits high ionic conductivity within this temperature range. An ionic conductivity of at least 10⁻³ S/cm is desirable in order for satisfactory efficiency of a PEM to be achieved. The polyoxometalate salts described herein achieve both increased stability and ionic conductivity due to their stoichiometric structure, thereby providing a material which is used to produce PEMs which are more efficient and stable.

The organic-inorganic hybrid compounds described herein are insoluble in water and are thus resistant to leaching under fuel cell operating conditions that generally involve the formation and transport of water through one or more components of the device or the device as a whole. The compounds are soluble in organic solvents of different dielectric constants such as for example dimethyl formamide.

The organic-inorganic hybrid compounds can be synthesized through a reaction in which the conjugate anion of the stronger acid replaces the conjugate anion of a weaker acid. A convenient method for the synthesis of said ion pairs is to combine a heteropolyacid - a polyoxometalate anion that has its negative charge neutralized by a stoichiometric number of protons - with a salt of the organic cation that will combine with the polyoxometalate anion in a new ion pair. The counterion to the organic cation can be, but is not limited to, any of the following anions: chloride, bromide, iodide, tetrafluoroborate, hexafluorophosphate, trifluoroacetate, methylsulfate, trifluoromethanesulfonate or trifluoromethanesulfonimide. Such salts generally possess low melting points and are therefore known as ionic liquids. The type of anion generally determines the miscibility of the ionic liquid with water but does not influence the formation and composition of the ion pair if the condition is met that the anion is a conjugate ion of an acid that is weaker than the heteropolyacid used in the reaction. The metathesis reaction may be carried out through mixing of the reagents in pure form, in suspension or in solution. At least one reagent may in liquid form either as a pure compound or in solution or suspension. The reaction may require heating, ultrasonic mixing or other methods of activation.

The organic-inorganic hybrid salts may be prepared from water soluble as well as water insoluble ionic liquids. "Ionic liquid" refers to an ionic compound with a low melting point, for example below 100°C.

When the salt synthesis is carried out in the presence of less polar or non-polar organic solvents (in which the polyoxometalate acid must be soluble), water insoluble ionic liquids are preferred for a more efficient reaction.

The organic-inorganic hybrid compounds described herein may be prepared from the reaction of an ionic liquid with a heteropolyacid. The heteropolyacid may be silicotungstic acid or phosphotungstic acid, having the formulae H₄W₁₂SiO₄₀ and H₃PW₁₂O₄₀ respectively. Examples of some possible ionic liquids which may be reacted with either of the above heteropolyacids to produce suitable polyoxometalate orqanic-inorqanic hybrids are given in the table below.

The chemical structures of the DMIM, EMIM, TMIM, NBMP, THTDP and PIM cations are provided above.

| **Compound** | **Structure** |
|---|---|
| A | [DMIM]CI |
| B | [DMIM]Br |
| C | [DMIM]I |
| D | [DMIM]BF₄ |
| E | [DMIM]PF₆ |
| F | [DMIM]CO₂CF₃ |
| G | [DMIM]CF₃SO₃ |
| H | [DMIM]CH₃SO₄ |
| I | [DMIM]NS₂O₄C₂F₆ |
| J | [EMIM]CI |
| K | [EMIM]Br |
| L | [EMIM]I |
| M | [EMIM]BF₄ |
| N | [EMIM]PF₆ |
| O | [EMIM]CO₂CF₃ |
| P | [EMIM]CF₃SO₃ |
| Q | [EMIM]CH₃SO₄ |
| R | [EMIM]NS₂O₄C₂F₆ |
| S | [TMIM]CI |
| T | [TMIM]Br |
| U | [TMIM]I |
| V | [TMIM]BF₄ |
| W | [TMIM]PF₆ |
| X | [TMIM]CO₂CF₃ |
| Y | [TMIM]CF₃SO₃ |
| Z | [TMIM]CH₃SO₄ |
| AA | [TMIM]NS₂O₄C₂F₆ |
| AB | [NBMP]CI |
| AC | [NBMP]Br |
| AD | [NBMP]I |
| AE | [NBMP]BF₄ |
| AF | [NBMP]PF₆ |
| AG | [NBMP]CO₂CF₃ |
| AH | [NBMP]CF₃SO₃ |
| AI | [NBMP]CH₃SO₄ |
| AJ | [NBMP]NS₂O₄C₂F₆ |
| AK | [THTDP]CI |
| AL | [THTDP]Br |
| AM | [THTDP]I |
| AN | [THTDP]BF₄ |
| AO | [THTDP]PF₆ |
| AP | [THTDP]CO₂CF₃ |
| AQ | [THTDP]CF₃SO₃ |
| AR | [THTDP]CH₃SO₄ |
| AS | [THTDP]NS₂O₄C₂F₆ |
| AT | [PIM]CI |
| AU | [PIM]Br |
| AV | [PIM]I |
| AW | [PIM]BF₄ |
| AX | [PIM]PF₆ |
| AY | [PIM]CO₂CF₃ |
| AZ | [PIM]CH₃SO₃ |
| BA | [PIM]CH₃SO₄ |
| BB | [PIM]NS₂O₄C₂F₆ |

((NS₂O₄C₂F₆)⁻ represents the bis(trifluoromethanesulfonyl)amide anion.

Thus the following organic-inorganic hybrid compounds are some of the possible compounds which may be formed in the method of the present invention.

| **Compound** | **Structure** |
|---|---|
| 1 | [DMIM]₄W₁₂SiQ₄₀ |
| 2 | [DMIM]₃PW₁₂O₄₀ |
| 3 | [EMIM]₄W₁₂SiQ₄₀ |
| 4 | [EMIM]₃PW₁₂O₄₀ |
| 5 | [TMIM]₄W₁₂SiO₄₀ |
| 6 | [TMIM]₃PW₁₂O₄₀ |
| 7 | [NBMP]₄W₁₂SiO₄₀ |
| 8 | [NBMP]₃PW₁₂O₄₀ |
| 9 | [THTDP]₄W₁₂SiO₄₀ |
| 10 | [THTDP]₃PW₁₂O₄₀ |
| 11 | [PIM]₄W₁₂SiO₄₀ |
| 12 | [PIM]₃PW₁₂O₄₀ |

The organic-inorganic hybrid compounds described herein undergo a phase-transition associated with a large step up in conductivity. This is observed for salts prepared by methods which use both soluble and insoluble ionic liquids as reactant.

The melting points of the organic-inorganic hybrid compounds according to the present invention fall within a wide range from 50°C to 400°C. Thus liquid electrolytes may be used in the high temperature fuel cells, e.g. in through-flow or falling film configurations.

Above the phase transition, the ionic conductivity of the organic-inorganic hybrid compounds is between 1 and 40 mS/cm, i.e. at least 10⁻³ S/cm. As explained above, such a high conductivity allows for satisfactory performance of the compounds as a component of a proton conducting electrolyte such as a proton exchange membrane.

The conductivity through finite dimensions of a macroscopic nanocomposite membrane sample is subject to reaching the percolation threshold, which is approximately 67 % (salt content). At and above the threshold salt content the salt crystallites are in sufficiently close proximity to act as channels of ionic conductivity through the macroscopic sample, unless the other membrane components provide the suitable links for proton conduction. For example, the nanocomposite may self-assemble in such a way as to form proton-conductivity channels.

The organic-inorganic hybrid compounds prepared according to the described method having the general properties described above may be a liquid or a solid at room temperature and have suitable properties that allows it to act as the proton conducting component in blends or precursors that may be used to manufacture proton exchange membranes (PEM). The precursors contain one or more ion-conducting or non-conducting polymers or copolymers able to withstand on a long term basis the operating conditions of the electrochemical device in which they are applied and impart the membrane with properties conducive to the intended use of the membrane and its production. High-performance chemically resistant, organic polymers that may be fluorinated and may contain acidic or basic functional groups may be used in this function. Examples of polymers that may be used for the preparation of PEM include high-performance polymers such as polysulfone, polyethersulfone, polyphenylenesulfone, polyimide, polyimideamide, polyetherimide, polyetheretherketone, polyvinylidene fluoride and sulfonated derivatives thereof.

### Combinations

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination.

The same is true for features described in the context of products and methods.

Any one or more of the aspects of the present invention may be combined with any one or more of the other aspects of the present invention. Similarly, any one or more of the features and optional features of any of the aspects may be applied to any one of the other aspects. Thus, the discussion herein of optional and preferred features may apply to some or all of the aspects. Furthermore, optional and preferred features associated with a method or use may also apply to a product and vice versa. For example, optional and preferred features relating to the organic-inorganic hybrid compound, precursor composition, or nanocomposite proton-exchange membrane, methods of making the organic-inorganic hybrid compound, precursor composition, or nanocomposite proton- exchange membrane and methods of using the organic-inorganic hybrid compound, precursor composition, or nanocomposite proton-exchange membrane, etc apply to all of the other aspects. Furthermore, optional and preferred features associated with a method or use may also apply to a product (e.g. organic-inorganic hybrid compound, precursor composition, or nanocomposite proton-exchange membrane) and vice versa.

The options, features, preferences and so on mentioned herein apply both independently and in any combination, except where such a combination is expressly prohibited or clearly impermissible.

### Examples

### Polyoxometalate Salt Preparation

### Example 1

A salt was synthesized by mixing aqueous solutions A and B. Solution A was obtained by dissolving 10 g of silicotungstic acid in 50 ml distilled water, solution B was obtained by dissolving 1.21 g of 1- butyl-3-methyl imidazolium chloride ([BMIM][CI]) and 1.71 g of 1,3-dimethylimidazol trifluoromethane sulfonate in 50 ml distilled water. The aqueous solution of ionic liquids was added into the aqueous solution of silicotungstic acid with automatic dosing pump (LAB PUMP Jr. Model RHSY, Fluid Metering Inc., Syosset, NY, USA) during 45 minutes mixing the solution. A white precipitate was formed. The suspension was mixed after the precipitation was completed for another 60 minutes. The precipitate was filtered and rinsed thoroughly with distilled water and then dried at 80°C in an oven overnight. 9.86 g of a crystalline solid was obtained which represents 90.07% of theoretical stoichiometric salt yield. The solid was characterized to have the composition SiWA-4(BMIM) + SiWA-4(DMIM). The obtained salt is soluble in N-methyl-2-pyrrolidone and imidazole mixture.

### Example 1A

A salt was synthesized by mixing aqueous solutions A and B. Solution A was obtained by dissolving 10 g of phosphotungstic acid in 50 ml distilled water, solution B was obtained by dissolving 1.82 g of 1-butyl-3-methyl imidazolium chloride ([BMIM][CI]) in distilled water. Immediately upon mixing a white precipitate was formed. The precipitate was filtered and rinsed thoroughly with distilled water, and 9.55 g of a crystalline solid was obtained. The solid was characterized to have the composition PWA-3(BMIM), i.e. [BMIM]₃[PW₁₂O₄₀].

### Example 1B

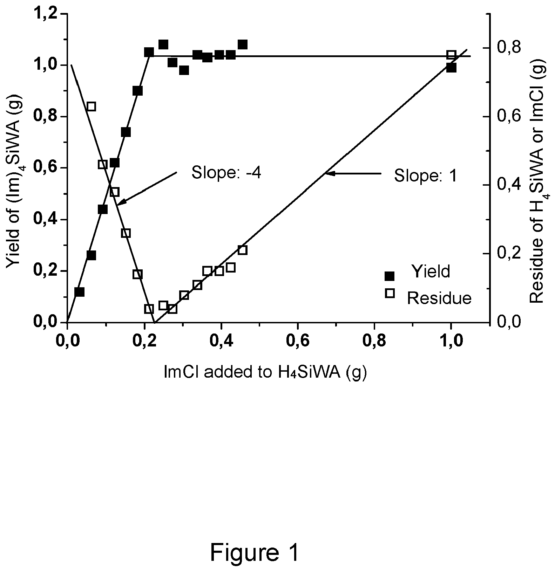

A series of salts was synthesized by carefully mixing aqueous solutions of silicotungstic acid (SiWA) and 1-butyl-3-methyl imidazolium chloride ([BMIM][CI]). Two aqueous solutions were prepared: solution A containing SiWA in a concentration of around 7 × 10⁻² mol/L, and solution B containing BMIMCI of a concentration of around 0.3 mol/L. Solutions A and B were mixed in such a way as to combine SiWA and BMIMCI in molar ratios between 1 : 0.5 and 1 : 7.5. The obtained solid precipitate from each reaction was isolated, thoroughly rinsed, dried to constant mass at 105°C and weighed. The analysis showed as a plot in Fig 1 demonstrates that the precipitate formed has a stoichiometric ratio of one SiWA anion (i.e. [W₁₂SiO₄₀]⁴⁺) combined with exactly four BMIM cations regardless of the reagent ratio used.

### Example 2

A salt was synthesized by mixing aqueous solutions A and B. Solution A was obtained by dissolving 10.63 g of phosphotungstic acid in 50 ml distilled water, solution B was obtained by dissolving 0.82 g of 1-butyl-3-methyl imidazolium chloride ([BMIM][CI]) and 1.15 g of 1,3-dimethylimidazol trifluoromethane sulfonate in 50 ml distilled water. The aqueous solution of ionic liquids was added into the aqueous solution of silicotungstic acid with automatic dosing pump (LAB PUMP Jr. Model RHSY, Fluid Metering Inc., Syosset, NY, USA) during 45 minutes mixing the solution. A white precipitate was formed. The suspension was mixed after the precipitation was completed for another 60 minutes. The precipitate was filtered and rinsed thoroughly with distilled water and then dried at 80°C in an oven overnight. 11.14 g of a crystalline solid was obtained which represents 93.0% of theoretical stoichiometric salt yield. The solid was characterized to have the composition PWA-3(BMIM) + PWA-3(DMIM). The obtained salt is soluble in N-methyl-2-pyrrolidone and imidazole mixture.

### Example 3

A salt was synthesized by mixing aqueous solutions A and B. Solution A was obtained by dissolving 3.0 g of phosphotungstic acid (PWA) in 100 ml distilled water and solution B was obtained by dissolving 0.38 g of 1,2,3-trimethylimidazolium iodide ([TMIM][I]) in 110 ml distilled water. The aqueous solution of ionic liquids was slowly added to the aqueous solution of phosphotungstic acid during 60 minutes while continuously mixing the solution. A white precipitate was formed. After the addition was completed the suspension was mixed for another 120 minutes. The precipitate was filtered and rinsed thoroughly with distilled water and then dried at 80°C in an oven overnight. 2.90 g of a crystalline solid was obtained. The solid was characterized to have the composition PWA- 3(TMIM). The obtained salt is soluble in an acetone/water mixture. Recrystallization of the salt from this solution gives monocrystals.

### Example 4

A salt was synthesized by mixing aqueous solutions A and B. Solution A was obtained by dissolving 3.0 g of silicotungstic acid (SiWA) in 100 ml distilled water and solution B was obtained by dissolving 0.50 g of 1,2,3-trimethylimidazolium iodide ([TMIM][I]) in 110 ml distilled water. The aqueous solution of ionic liquids was slowly added to the aqueous solution of silicotungstic acid during 60 minutes while continuously mixing the solution. A white precipitate was formed. After the addition was completed the suspension was mixed for another 120 minutes. The precipitate was filtered and rinsed thoroughly with distilled water and then dried at 80°C in an oven overnight. 1.62 g of a crystalline solid was obtained, which represents 81.0% of theoretical yield. The solid was characterized to have the composition SiWA-4(TMIM). The obtained salt is soluble in an acetonitrile/acetone/water mixture.

### Example 5

A salt was synthesized by mixing aqueous solutions A and B. Solution A was obtained by dissolving 5 g of phosphotungstic acid in 25 ml distilled water and solution B was obtained by dissolving 1.71 g of 1,3-dimethyl imidazolium trifluoromethanesulfonate ([DMIM][F₃CSO₃]) in 25 ml distilled water. The aqueous solution of ionic liquids was slowly added to the aqueous solution of phosphotungstic acid during 60 minutes while continuously mixing the solution. A white precipitate was formed. After the addition was completed the suspension was mixed for another 120 minutes. The precipitate was filtered and rinsed thoroughly with distilled water and then dried at 80°C in an oven overnight. 5.62 g of a crystalline solid was obtained which represents 96.07% of theoretical stoichiometric salt yield. The solid was characterized to have the composition PWA-3(DMIM). The obtained salt is soluble in N-Ethyl-2-pyrrolidone.

### Example 6

A salt was synthesized by mixing aqueous solutions A and B. Solution A was obtained by dissolving 5g of silicotungstic acid in 25 ml distilled water and solution B was obtained by dissolving 2.28 g of 1,3- dimethyl imidazolium trifluoromethanesulfonate ([DMIM][F₃CSO₃]) in 25 ml distilled water. The aqueous solution of ionic liquids was slowly added to the aqueous solution of silicotungstic acid during 60 minutes while continuously mixing the solution. A white precipitate was formed. After the addition was completed the suspension was mixed for another 120 minutes. The precipitate was filtered and rinsed thoroughly with distilled water and then dried at 80°C in an oven overnight. 5.92 g of a crystalline solid was obtained which represents 96.4% of theoretical stoichiometric salt yield. The solid was characterized to have the composition SiWA-4(DMIM). The obtained salt is soluble in N- Ethyl-2-pyrrolidone (NEP).

### Example 7

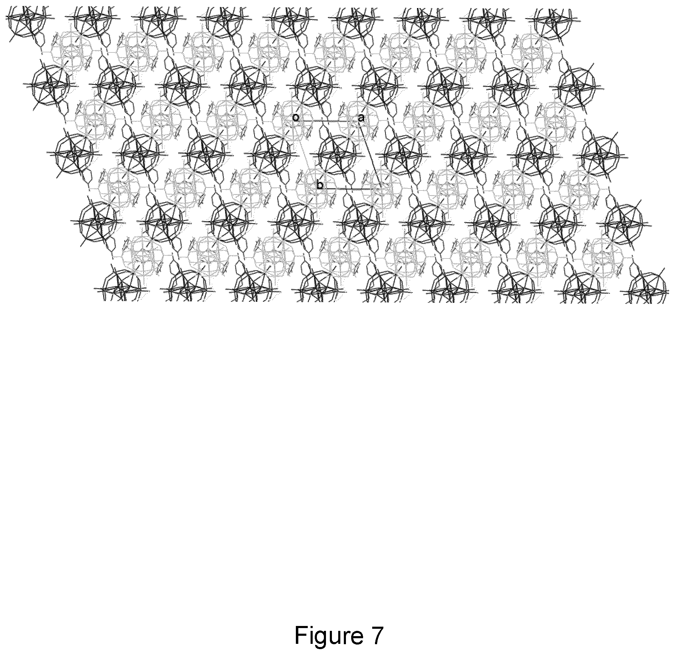

A salt was synthesized by mixing an aqueous solution of phosphotungstic acid and water-insoluble N- butyl-4-methylpyridinium hexaflurophosphate ([NBMP][PF₆]). 3 g of phosphotungstic acid was dissolved in 50 ml of distilled water and 1.15 g of N-butyl-4-methylpyridinium hexaflurophosphate was added into solution during mixing. A white precipitate was formed. The suspension was mixed after the precipitation was completed for another 120 minutes. The precipitate was filtered and rinsed thoroughly with distilled water and then dried at 80°C in an oven overnight. 3.2 g of a crystalline solid was obtained which represents 90.14% of theoretical stoichiometric salt yield. The solid was characterized to have the composition PWA-3(NBMP). The obtained salt is soluble in acetonitrile and insoluble in water. Recrystallization of the salt in acetonitrile yields monocrystals. The structure and elemental composition of the salt obtained with single crystal X-ray diffraction is given in Table 1 below and Figs 7a, 7b and Fig 8.

**Table 1 - Single crystal X-ray structure determination of salt**

| K1529 (N-Bu-4-Me-Py)₃PW₁₂O₄₀ Sample designation 7P | |
|---|---|
| Assymmetric unit formula | C30H4sN3O40P1W12 |
| Assymmetric unit relative molecular mass | 3327.87 |
| Unit cell relative molecular mass | 6655.74 |
| Unit cell composition | CsoH9sNsOsoP2W24 |
| Unit cell parameters | a= 11.7003(2) A |
| | *b* = 12.5725(2) A |
| | c = 22.6699(3) A |
| | *a*= 81.8275(11)° |
| | *β* = 75.5739(11)° |
| | *y* = 69.2714(10) |
| | V= 3015.21(8)A³ |
| Crystal class and space group | triclinic, *P*-1 |
| Elemental composition of unit cell (wt.%) | 10.83 %C, |
| | 1.45%H, |
| | 1.26 % N, |
| | 19.23 % 0, |
| | 0.93 % P, |
| | 66.29 % W |
| Cation mass fraction | 13.54 % |

### Example 8

A salt was synthesized by mixing an aqueous solution of silicotungstic acid and water-insoluble N- butyl-4-methylpyridinium hexaflurophosphate. 3.0 g of silicootungstic acid was dissolved in 50 ml of distilled water and 1.54 grams of N-butyl-4-methylpyridinium hexaflurophosphate ([NBMP][PF₆]) was added into solution during mixing. A white precipitate was formed. The suspension was mixed after the precipitation was completed for another 120 minutes. The precipitate was filtered and rinsed thoroughly with distilled water and then dried at 80°C in an oven overnight. 3.30 g of a crystalline solid was obtained which represents 87.75% of theoretical stoichiometric salt yield. The solid was characterized to have the composition SiWA-4(NBMP). The obtained salt is soluble in acetonitrile and insoluble in water. Recrystallization of the salt in acetonitrile yields monocrystals.

### Example 9

A salt was synthesized by mixing aqueous solutions A and B. Solution A was obtained by dissolving 5 g of silicotungstic acid in 25 ml distilled water and solution B was obtained by dissolving 1.35 g of 1- ethyl-3-methyl imidazolium methylsulfate ([EMIM][CH₃O₄S⁻]) in 25 ml distilled water. The aqueous solution of ionic liquids was slowly added to the aqueous solution of silicotungstic acid during 60 minutes while continuously mixing the solution. A white precipitate was formed. After the addition was completed the suspension was mixed for another 120 minutes. The precipitate was filtered and rinsed thoroughly with distilled water and then dried at 80°C in oven overnight. 4.93 g of a crystalline solid was obtained which represents 77.63% of theoretical stoichiometric salt yield. The solid was characterized to have the composition SiWA-4(EMIM). The obtained salt is soluble in dimethylformamide (DMF).

### Example 10

A salt was synthesized by mixing an aqueous solution of 1.01 g phosphotungstic acid and 0.57 grams of trihexyltetradecylphosphonium chloride ([THTDP][CI]) (molar ratio acid : IL= 1 : 3) in 7 ml of distilled water at room temperature. The solution was sonicated at 37 kHz and room temperature for 75 minutes. After that it was cooled to -5°C for 5 hours. The precipitate was then kept overnight at room temperature and then again sonicated at 80 kHz for 2 hours and cooled for 3 hours at -7°C. Finally, the precipitate was kept at room temperature overnight and rinsed with distilled water until the pH of water over the precipitate reached 5.5. The water was decanted and the precipitate dried at 80°C overnight and cooled to room temperature. 1.48 g of a white salt was obtained, which represents 99.3% of theoretical yield. The salt exhibits typical ionic liquid behaviour with a melting point below 85°C.

### Example 11

A salt was synthesized by mixing an aqueous solution of 1.00 grams silicotungstic acid and 0.73 grams of trihexyltetradecylphosphonium chloride ([THTDP][CI]) (molar ratio acid : IL= 1 : 4) in 7 ml of distilled water at room temperature. The solution was sonicated at 37 kHz and room temperature for 75 minutes. After that it was cooled to -5°C for 5 hours. The precipitate was kept overnight at room temperature and then again sonicated at 80 kHz for 2 hours and cooled for 3 hours at -7°C. Finally, the precipitate was kept at room temperature overnight and rinsed with distilled water until the pH of water over the precipitate reached 5.5. The water was decanted and the precipitate dried at 80°C overnight and cooled to room temperature. 1.28 g of a white salt was obtained, which represents 99.6% of theoretical yield. The salt exhibited typical ionic liquid behavior with a melting point below 60 °c.

### Example 12

A salt was synthesized by mixing aqueous solution of 1.00 g phosphotungstic acid and 0.80 g of trihexyltetradecylphosphonium bis(trifluoromethylsulfonyl) amide ([THZDP][N(SO₂⁻CF₃)₂]) (molar ratio acid : IL= 1 : 3) in 7 ml of distilled water at room temperature. The solution was sonicated at 37 kHz and room temperature for 75 minutes. After that it was cooled to -5°C for 5 hours. The precipitate was then kept overnight at room temperature and then again sonicated at 80 kHz for 2 hours and cooled for 3 hours at -7°C. Finally, the precipitate was kept at room temperature overnight and rinsed with distilled water until the pH of water over the precipitate reached 5.5. The water was decanted and the precipitate dried at 80°C overnight and cooled to room temperature. 1.44 g of a pale yellow salt was obtained, which represents 90.2 % of theoretical yield. This salt exhibits typical ionic liquid behaviour with a melting point below 75°C.

### Example 13

A salt was synthesized by mixing aqueous solutions A and B. Solution A was obtained by dissolving 3g of silicotungstic acid in 60 ml distilled water and solution B was obtained by dissolving 1.5 g of polyimidazolium bromide (PIB) in 30 ml distilled water. The aqueous solution of ionic liquid was slowly added to the aqueous solution of silicotungstic acid during 60 minutes while continuously mixing the solution. A white precipitate was formed. After the addition was completed the suspension was mixed for another 120 minutes. The precipitate was filtered and rinsed thoroughly with distilled water and then dried at 80°C in an oven overnight. 4.3 g of a crystalline solid was obtained which represents 95.56% of theoretical stoichiometric salt yield. The solid was characterized to have the composition SiWA-4(PIB). The obtained salt is soluble in low concentrations in N-methyl-2- pyrrolidone (NMP).

### Example 14

An ion pair was produced by the method according to the present invention and had a stoichiometric composition where the combined charge of the organic cations matches exactly the charge of the polyoxometalate anion. A detailed and systematic quantitative study of the ion pairs produced by this method showed that regardless of excess of one reagent a single, stoichiometric cation vs. anion ratio is formed for a given organic cation - polyoxometalate anion ion pair. Fig 1 demonstrates one example showing the mass balance of the ion exchange reaction between silicotungstic acid and an imidazolium chloride ionic liquid at different reagent ratios.

### Example 15

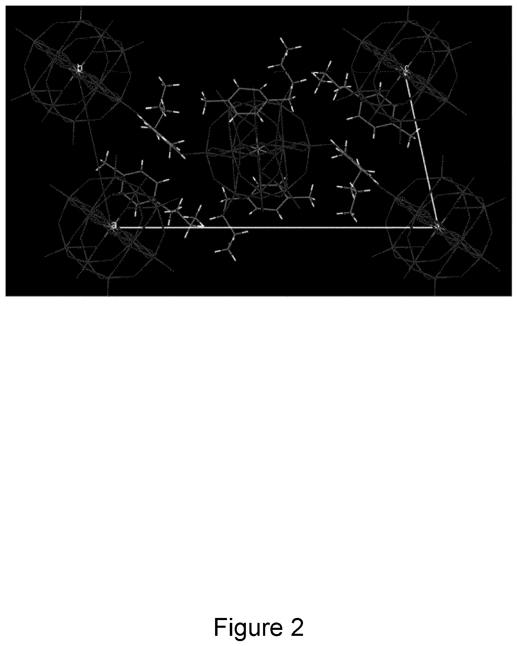

A purified organic cation - polyoxometalate anion ion pair according to the present invention was prepared in the form of a single crystal. X-ray diffraction analysis of the structures confirmed an exact stoichiometric composition of the ion pair. Fig 2 shows one example of the single crystal structure of an ion pair formed by the reaction of phosphotungstic acid hydrate and 1-butyl-3-methyl imidazolium hexafluorophosphate (BMIMPF₆).

### Example 16

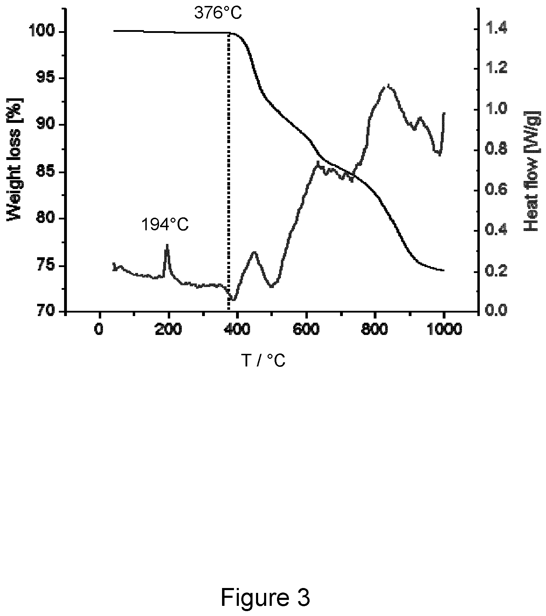

The ion pairs prepared by the method according to the present invention are insoluble in water and are highly stable. They may be in the form of liquids or solids. The ion pairs are not hygroscopic. Thermal gravimetric analysis has revealed that the said ion pairs prepared by the methods outlined in this disclosure have high thermal stability with a minor loss of weight up to temperatures 300°C or more. Fig 3 shows a thermogram giving a representative illustration of said characteristics. The described thermal behavior also shows that the ion pair, made by reacting silicotungstic acid and BMIMCI, is not in hydrated form at ambient conditions, indicating that the transition from a heteropolyacid to an organic cation - polyoxometalate anion ion pair radically alters the affinity of the material towards water.

### Example 17

Impedance spectroscopy measurements were used to investigate the special ionic conductivity properties of the ion pairs according to the present invention which are intrinsically linked to their chemical structure, and have shown that the ion pair exhibits low ionic conductivity up to the order 10⁻⁶ S/cm up to a temperature where a phase transition occurs. This transition coincides with a rapid increase in ionic conductivity by several orders of magnitude. Further heating above the transition temperature causes the ionic conductivity to increase further albeit at a much lower rate. Upon cooling the ionic conductivity is reduced in a reversible way although the conductivity transition cycle may exhibit a hysteresis, with the cooling phase transition occurring at a temperature lower than the temperature of the phase transition during heating.

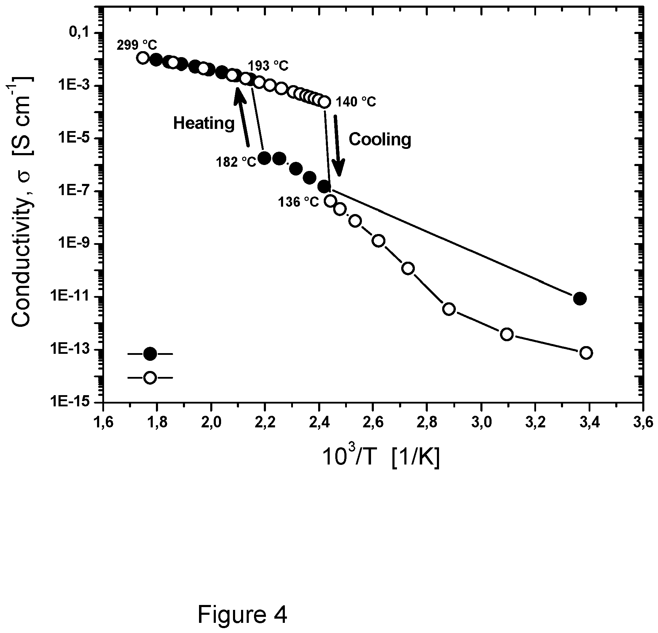

Fig 4 shows a representative conductivity vs. reciprocal temperature diagram obtained from impedance spectroscopy measurement in dry helium of an ion pair prepared by the reaction of silicotungstic acid with BMIMCI and exhibiting the above-described dependence of ionic conductivity on reciprocal temperature, including the hysteresis effect. The example shows that the phase transition occurs at 182°C, where an increase in the ionic conductivity by approximately three orders of magnitude takes place. A high ionic conductivity on the order of 10⁻² S/cm was achieved at temperatures up to 299°C. Upon cooling the phase transition temperature is 140°C. This example illustrates that the organic cation - polyoxometalate anion ion pair is capable of sustaining ionic conductivity above 10⁻³ S/cm in the temperature range 195-300°C without the presence of water.

### Example 18

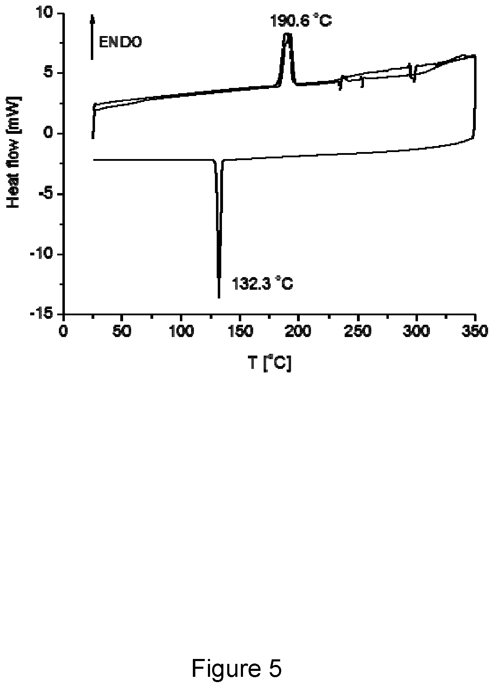

The phase transition at which the ionic conductivity of the ion pair formed by the reaction of silicotungstic acid and BMIMCI containing a polyoxometalate anion undergoes a significant change in ionic conductivity coincides with a thermal absorption peak that can be detected by differential scanning calorimetry (DSC). A representative DSC thermogram is shown in Fig 5. An endothermic peak occurs at 190.6°C in the heating scan, which corresponds exactly with the ionic conductivity jump observed by impedance spectroscopy. The exothermic peak in the cooling scan occurs at 132.3°C. Heat flows for the endothermic and exothermic peaks are equal in magnitude confirming that this is a reversible transition. Consecutive heating scans show that the transition is repeatable.

The peaks observed in the DSC thermograms resemble a thermogram trace resulting from melting however inspection of a sample on a melting point apparatus during heating revealed that melting does not occur at the temperature where the transition takes place as observed by the thermal analytical methods listed above. These results indicate that the described ionic pairs undergo an order-disorder phase transition.

## Claims

1. A process for preparing an organic-inorganic hybrid compound comprising the step of mixing a heteropolyacid with an ionic liquid comprising an organic cation and a counter anion to perform a metathesis reaction,
wherein
(i) the organic-inorganic hybrid compound formed comprises a stoichiometric ratio of organic cation to heteropolyoxometalate anion;
(ii) the organic cation is selected from 1,3-dimethyllimidazolium (DMIM), 1-ethyl-3-methylimidazolium (EMIM), 1,2,3-trimethylimidazolium (TMIM), N-butyl-4-methylpyridinium (NBMP), trihexyltetradecylphosphonium (THTDP) and polyimidazolium (PIM), wherein the number of repeat units in polyimidazolium is 5 to 1000; and
(iii) the heteropolyoxometalate anion is selected from silicotungstate and phosphotungstate.

2. A process according to claim 1, further comprising the step of washing the reaction product with a washing solvent in which the organic-inorganic hybrid compound is insoluble, but in which other species present in the reaction mixture are soluble.

3. A process according to claim 2, wherein the washing solvent is water.

4. A process according to any one of claims 1 to 3, wherein the further step of recrystallization of the organic-inorganic hybrid compound is performed.

5. A process according to any one of claims 1 to 4, wherein the counter anion in the ionic liquid is chloride, bromide, iodide, tetrafluoroborate, hexafluorophosphate, trifluoroacetate, methylsulfate, trifluoromethanesulfonate or trifluoromethanesulfonimide.

6. A process according to any one of claims 1 to 5, wherein the ionic liquid is insoluble in water.

## Patentansprüche

1. Verfahren zur Herstellung einer organisch-anorganischen Hybridverbindung, das den Schritt des Vermischens einer Heteropolysäure mit einer ionischen Flüssigkeit umfasst, die ein organisches Kation und ein Gegenanion umfasst, um eine Metathesereaktion durchzuführen, wobei:
(i) die gebildete organisch-anorganische Hybridverbindung ein stöchiometrisches Verhältnis zwischen dem organischen Kation und einem Heteropolyoxometalat-anion aufweist;
(ii) das organische Kation aus 1,3-Dimethyllimidazolium (DMIM), 1-Ethyl-3-Methyl-imidazolium (EMIM), 1,2,3-Trimethylimidazolium (TMIM), N-Butyl-4-methylpyridinium (NBMP), Trihexyltetradecylphosphonium (THTDP) und Polyimidazolium (PIM), wobei die Anzahl an Repetiereinheiten im Polyimidazolium 5 bis 1000 beträgt, ausgewählt ist; und
(iii) das Heteropolyoxometalat-Anion aus Silicowolframat und Phosphowolframat ausgewählt ist.

2. Verfahren nach Anspruch 1, das weiters den Schritt des Waschens des Reaktionsprodukts mit einem Waschlösungsmittel umfasst, in dem die organisch-anorganische Hybridverbindung unlöslich ist, in dem aber andere im Reaktionsgemisch vorhandene Spezies löslich sind.

3. Verfahren nach Anspruch 2, wobei das Waschlösungsmittel Wasser ist.

4. Verfahren nach einem der Ansprüche 1 bis 3, wobei ein weiterer Schritt einer Umkristallisation der organisch-anorganischen Hybridverbindung durchgeführt wird.

5. Verfahren nach einem der Ansprüche 1 bis 4, wobei das Gegenanion in der ionischen Flüssigkeit Chlorid, Bromid, lodid, Tetrafluoroborat, Hexafluorophosphat, Trifluoracetat, Methylsulfat, Trifluormethansulfonat oder Trifluormethansulfonimid ist.

6. Verfahren nach einem der Ansprüche 1 bis 5, wobei die ionische Flüssigkeit in Wasser unlöslich ist.

## Revendications

1. Procédé de préparation d'un composé hybride organique-inorganique comprenant l'étape de mélange d'un hétéropolyacide avec un liquide ionique comprenant un cation organique et un contre-anion pour effectuer une réaction de métathèse,
dans lequel
(i) le composé hybride organique-inorganique formé comprend un rapport stoechiométrique entre un cation organique et un anion hétéropolyoxométalate ;
(ii) le cation organique est choisi parmi les 1,3-diméthyllimidazolium (DMIM), 1-éthyl-3-méthylimidazolium (EMIM), 1,2,3-triméthylimidazolium (TMIM), N-butyl-4-méthylpyridinium (NBMP), trihexyltétradécylphosphonium (THTDP) et polyimidazolium (PIM), dans lequel le nombre d'unités répétitives dans le polyimidazolium est de 5 à 1 000 ; et
(iii) l'anion hétéropolyoxométalate est choisi parmi du silicotungstate et du phosphotungstate.

2. Procédé selon la revendication 1, comprenant en outre l'étape de lavage du produit de réaction avec un solvant de lavage dans lequel le composé hybride organique-inorganique est insoluble, mais dans lequel d'autres espèces présentes dans le mélange de réaction sont solubles.

3. Procédé selon la revendication 2, dans lequel le solvant de lavage est l'eau.

4. Procédé selon l'une quelconque des revendications 1 à 3, dans lequel l'étape supplémentaire de recristallisation du composé hybride organique-inorganique est effectuée.

5. Procédé selon l'une quelconque des revendications 1 à 4, dans lequel le contre-anion dans le liquide ionique est un chlorure, bromure, iodure, tétrafluoroborate, hexafluorophosphate, trifluoroacétate, méthylsulfate, trifluorométhanesulfonate ou trifluorométhanesulfonimide.

6. Procédé selon l'une quelconque des revendications 1 à 5, dans lequel le liquide ionique est insoluble dans l'eau.
